# EUROPEAN PATENT SPECIFICATION

(11) **EP 4 010 073 B1**
(45) Date of publication and mention of the grant of the patent: **26.02.2025**
(21) Application number: 20754205.1
(22) Date of filing: 05.08.2020
(51) Int. Cl.: A61N 5/10

(54) **METHOD FOR USE WITH A RADIOTHERAPY DEVICE**
VERFAHREN ZUR VERWENDUNG MIT EINER STRAHLENTHERAPIEVORRICHTUNG
PROCÉDÉ DESTINÉ À ÊTRE UTILISÉ AVEC UN DISPOSITIF DE RADIOTHÉRAPIE

(30) Priority: 05.08.2019 GB 201911178
(43) Date of publication of application: 15.06.2022
(73) Proprietor: Elekta Limited, Crawley RH10 9RR (GB)
(72) Inventor: FLINT, Chris, Crawley RH10 9RR (GB); VEGA, German, Crawley RH10 9RR (GB); RICHARDSON, Keith, Crawley RH10 9RR (GB); CHIAP, Alessandra, Crawley RH10 9RR (GB)
(74) Representative: St Clair Jones, Gregory Arthur Langley
(86) International application number: PCT/EP2020/072066
(87) International publication number: WO 2021/023797

(56) References cited:
- EP-A2- 1 454 657
- WO-A1-90/12413
- SONG YOUNG-GI: "Interlock system for machine protection of the KOMAC 100-MeV proton linac", JOURNAL OF THE KOREAN PHYSICAL SOCIETY, KOREAN PHYSICAL SOCIETY, KR, vol. 66, no. 3, 22 February 2015 (2015-02-22), pages 449 - 453, XP035455003, ISSN: 0374-4884, [retrieved on 20150222], DOI: 10.3938/JKPS.66.449

## Description

This disclosure relates to the field of remote diagnostics, and in particular to a method of identifying the nature of a fault in a radiotherapy device performing sub-optimally.

### Background

Radiotherapy devices are an important tool in modern cancer treatment. Radiotherapy devices are large, complex machines, with many moving parts and inter-operating mechanisms. Despite precision engineering and rigorous testing, some component parts of a radiotherapy machines may start to degrade over the lifetime of the machine. This can sometimes lead to sub-optimal operation and even the occasional safety override.

If at any point during treatment a radiotherapy device starts to function outside of its normal operating parameters, a safety override or "interrupt" occurs, whereby the machine stops delivering radiation to ensure patient safety. Such an event is inconvenient, as it adds time to the treatment, and in some cases means the treatment session must finish prematurely. Unplanned equipment downtime can disrupt planned treatment schedules, and may be expensive for the owner, be it due to loss of revenue, servicing and repair costs, or both.

It has been surmised that, as with other industries, predictive maintenance and/or remote diagnostic techniques could be applied to radiotherapy machines. However, given the complexity of the machines and the sheer volume of data which may be gathered during operation, it is difficult to know how to analyse any available data to inform the predictive maintenance techniques. For example, while particular data patterns may be indicative of a particular fault, identifying the link between particular data patterns and the particular fault is often non-intuitive even for experienced service engineers. Even when a problematic machine is identified, trying to ascertain the nature of the fault is very difficult given the abundance of data and the complex interrelationships between the various components of the machine. In other words, even if a wealth of data from a radiotherapy device is available, successfully determining the nature of a fault is not a trivial matter.

The present invention seeks to address these and other disadvantages encountered in the prior art by providing a method of identifying, preferably remotely, the nature of a fault in a radiotherapy device, for example a device which is performing sub-optimally.

EP 1454657A2 describes a particle beam therapy system which transports an ion beam emitted from an accelerator to one of a plurality of treatment rooms.

WO9012413 describes a raster scan control system for use with a charged-particle beam delivery system which provides control of large currents driving an inductive load.

### Summary

An invention is set out in the independent claims. Optional features are set out in the dependent claims.

### Figures

Specific embodiments are now described, by way of example only, with reference to the drawings, in which:
figure 1 depicts a schematic illustration of a LINAC device;
figure 2 depicts a cross-section through the vacuum tube of the LINAC device of figure 1;
figure 3 depicts a bending magnet assembly according to the present disclosure;
figure 4 depicts a power supply scheme for a bending magnet assembly according to the present disclosure;
figure 5 depicts a graph showing data which may be used in methods of the present disclosure;
figure 6 depicts a method according to the present disclosure;
figure 7 depicts a method according to the present disclosure; and
figure 8 depicts a method according to the present disclosure.

### Detailed Description

The present disclosure relates to a method of determining the nature of a fault in a radiotherapy machine or device. The device may be suitable for delivering a beam of radiation to a patient in order to treat a tumour. An example of a radiation source for producing a beam is a linear accelerator (LINAC). Clinical LINAC devices are configured to deliver high energy radiation to a patient.

Radiotherapy machines are beginning to be configured to produce and record a large amount of data as they operate; for example radiotherapy machines are configured to provide sensor readings from a variety of different sensors. These sensors produce data which can be stored in a database. Radiotherapy devices may also be configured to allow remote connection, enabling service engineers to access a wealth of information about any connected machine without having to travel to the site where the machine is located. It is expected that, in many cases, machines may be returned to optimal performance without an engineer ever having to physically interact with the machine. However, there will still be occasions where the fault cannot be fixed remotely, and an engineer must be sent to: inspect the machine; determine the nature of the fault; and perform any maintenance required. If the repair involves replacing a part, further machine downtime is required before the machine can be brought back online.

The present methods involve evaluating the condition and/or performance of radiotherapy equipment during its operation in order to identify, preferably remotely, the nature of a fault in a radiotherapy device which is performing sub-optimally. Such techniques are advantageous as they allow a manufacturer or maintenance service provider to attend the machine knowing what will be required to fix the machine prior to arrival. The disclosed techniques can help to reduce machine downtime and thereby minimise disruption to the machine's normal operation. The disclosed techniques can also be used to more effectively plan machine downtime for times which are more convenient or cost-effective for the owner of the equipment and/or the patients.

One potential fault in a LINAC device relates to a malfunctioning of the bending magnet assembly of the LINAC. This may, for example be caused by reduced performance of the cooling apparatus for the bending magnet assembly. The cooling apparatus may experience a blockage, which may manifest itself gradually over time, or indeed suddenly, if some foreign body enters the cooling circuitry and blocks a cooling line, or a build up of such bodies occurs over time and results in reduced flow of cooling fluid. Alternatively, the bending magnet assembly may be subject to physical motion, as it forms part of a moveable gantry. This in turn can negatively impact beam quality. The results of blockages, or the effects of bending magnet assembly movement on the LINAC can be monitored as part of safety and quality control procedures to ensure that, if the bending magnet voltage or a temperature of the cooling system varies past a certain point, a fault is identified, or a safety override or interrupt occurs.

As will be discussed below, a potential cause of a reduced quality of bending magnet operation is a blockage in the cooling system. Such blockages may be caused, for example, by small foreign bodies entering the cooling system over time as the LINAC operates. Machines where the fault is of this nature have variable performance and are subject to unwanted treatment interruptions, and given the large amount of possible causes of a poorly performing bending magnet assembly it is extremely difficult to determine the nature of the fault. It is possible that it is not known that the cooling system is the cause of the machine operating sub-optimally. Machines with bending magnet assembly related faults often have machine output fluctuations, and so a field service engineer investigating the fault may incorrectly conclude that the sub-optimal performance of the machine is due to, for example, a magnetron issue, rather than due to a fault with the cooling system of the bending magnet assembly. The present disclosure advantageously allows a determination that the nature of the fault is associated with the bending magnet assembly.

### High-level overview of a LINAC

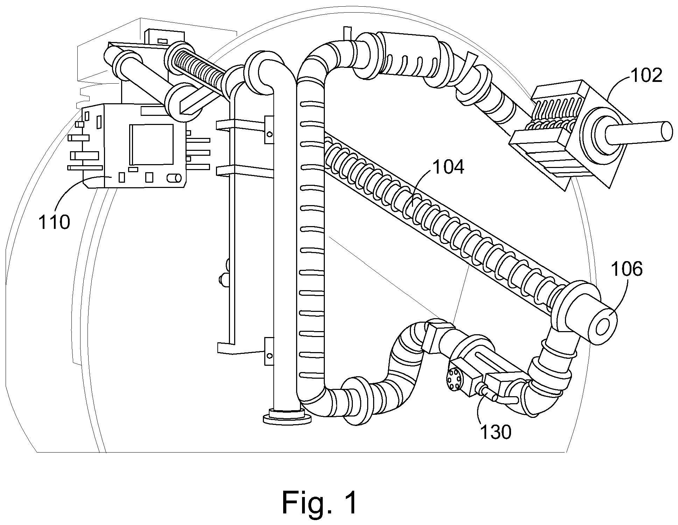

Figure 1 depicts a LINAC suitable for delivering, and configured to deliver, a beam of radiation to a patient during radiotherapy treatment. In operation, the LINAC device produces and shapes a beam of radiation and directs it toward a target region within the patient's body in accordance with a radiotherapy treatment plan.

A medical LINAC machine is by necessity complex, with many inter-operating component parts. A brief summary of the operation of a typical LINAC will be given with respect to the LINAC device depicted in figure 1, which comprises a source of radiofrequency waves , a waveguide, a source of electrons, a system capable of creating a strong vacuum comprising one or more vacuum pumps, a heavy metal target which produces X-rays when hit by an electron beam, and a complex arrangement of magnets capable of re-directing and focusing the electron beam onto the target. The device depicted in figure 1 also comprises a treatment head which houses various apparatus configured to, for example, collimate and shape the resultant X-ray beam.

The source 102 of radiofrequency waves, such as a magnetron, produces radiofrequency waves. The source 102 of radiofrequency waves is coupled to the waveguide 104, and is configured to pulse radiofrequency waves into the waveguide 104. Radiofrequency waves pass from the source 102 of radiofrequency waves through an RF input window and into a RF input connecting pipe or tube. The RF input connecting pipe or tube is coupled with the waveguide, and may join the waveguide at a substantially 90° angle as shown in figure 1. The connecting tube or pipe may join the waveguide via a so-called 'elbow joint' or 't-shaped joint'. A source 106 of electrons, such as an electron gun, is coupled to the waveguide 104 and is configured to inject electrons into the waveguide 104. In the source 106 of electrons, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source 106 and the waveguide 104 is such that the radiofrequency waves accelerate the electrons to very high energies as they propagate through the waveguide 104. The design of the waveguide 104 depends on whether the LINAC accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104.

As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they pass into a flight tube. The flight tube may be connected to the waveguide by a connecting tube. This connecting tube or connecting structure may be called a drift tube. The drift tube also forms part of the vacuum tube. RF waves exit the waveguide via an RF output connecting pipe or tube coupled with the drift tube. As with the RF input pipe which introduces RF to the waveguide, the pipe or tube through which RF exits the waveguide connects to the vacuum tube via an elbow joint or 'T-shaped' joint. RF passes out from the vacuum system via an RF output window which seals the vacuum system.

The flight tube is kept under vacuum conditions by the pump system 130. The electrons travel along a slalom path toward the heavy metal target. The target may comprise, for example, tungsten. Whilst the electrons travel through the flight tube, an arrangement of focusing magnets act to direct and focus the beam on the target. The slalom path allows the overall height of the LINAC to be reduced while ensuring that the beam of accelerated electrons, which is comprised of electrons with a small spread of energies, is focused on the target.

The slalom path of the electrons as they reach the end of the flight tube is created by a bending magnet assembly. The bending magnet assembly comprises a magnet array which provides focussing of the electron beam, which is described further below. Under the influence of the bending magnets, the beam is deflected from an angle above the horizontal to around 90° below, i.e. straight down at a target area on a patient. The path of the beam in the beam bending system depends on all of the following characteristics: field strengths of the individual bending magnets, the shape of the magnet poles, the beam energy, and the attitude of the beam on entry into the magnetic field. Further features of the bending magnet assembly are described below.

When the high energy electrons hit the target, X-rays are produced in a variety of directions, but mainly forwards. The target is located inside and/or part of the flight tube and makes up part of the vacuum system. X-rays which are produced when the LINAC is in operation pass through the target and into the treatment head 110. At this point, a primary collimator blocks X-rays travelling in certain directions and passes only forward travelling X-rays to produce a cone shaped beam. The X-rays are filtered, and then pass through one or more ion chambers for absolute dose and spatial intensity measurement. The beam can be shaped in various ways by beam-shaping apparatus, for example by using a multi-leaf collimator, before it passes into the patient as part of radiotherapy treatment.

In some implementations, the LINAC is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the LINAC. In essence, it is possible to swap between the first and second mode by moving the heavy metal target in or out of the electron beam path and replacing it with a so-called 'electron window'. The electron window is substantially transparent to electrons and allows electrons to exit the flight tube.

The LINAC device also comprises several other components and systems. The whole system is cooled by a water cooling system (not shown in the figures). The water cooling system may be used, in particular, to cool the waveguide 104, target, and radiofrequency source 102. In order to ensure the LINAC does not leak radiation, appropriate shielding is also provided. As will be understood by the person skilled in the art, a LINAC device used for radiotherapy treatment will have additional apparatus such as a gantry to support and rotate the LINAC, a patient support surface, and a controller or processor configured to control the LINAC apparatus.

### Details of apparatus and sensors - Case specific

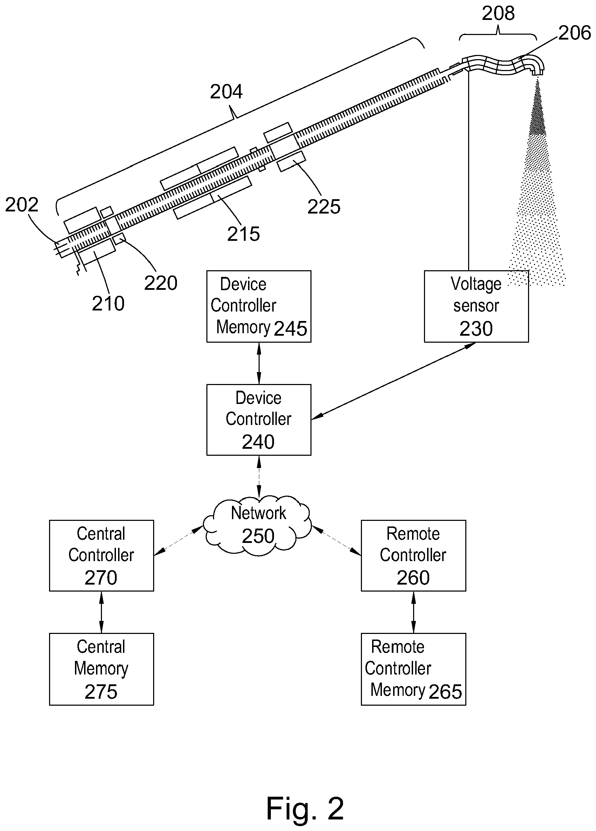

Figure 2 depicts a cross-section through a vacuum tube of a linac. As detailed above, the vacuum tube is comprised of an electron gun 202, a waveguide 204, and a flight tube 206. The flight tube is arranged to pass through a bending magnet assembly 208. The electron gun 202 is configured to inject electrons into the waveguide 204. In this example, the electron beam may be focused by a first arrangement of focusing magnets 210 and a second arrangement of focusing magnets 215. The beam is 'steered', i.e. directed, by a first arrangement of steering magnets 220 and a second arrangement of steering magnets 225. While the linac is in use, the electron gun 202, waveguide 204 and flight tube 206 are kept under high vacuum conditions by a vacuum system or suitable vacuum apparatus. The electron beam is focused and directed by the bending magnet assembly 208.

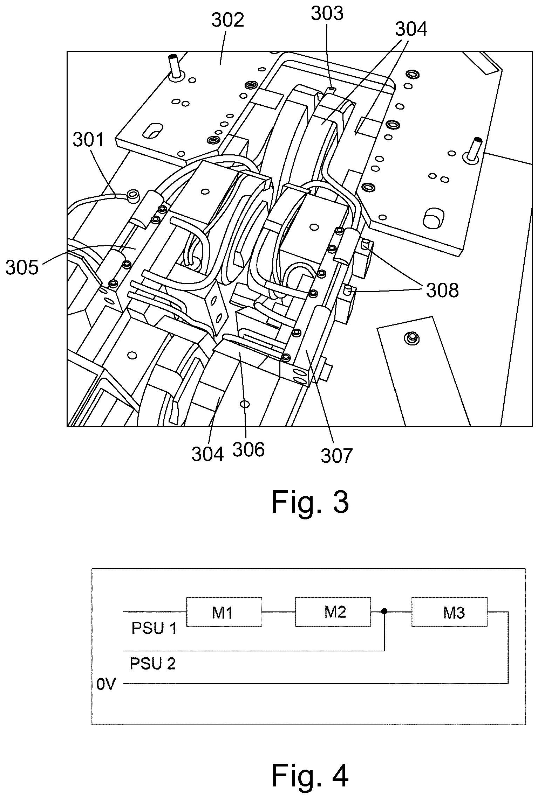

The bending magnet assembly 208 comprises a plurality of electromagnets arranged around the flight tube so as to bend the electron beam. The example shown in figure 3 comprises 3 electromagnet coils which combine to form an achromatic triple-magnet array. The evacuated flight tube contains the electron beam and is positioned between pole pieces of the bending magnet assembly.

The first magnet (M1), which may eb described as a bending coarse magnet, acts as an energy analyser and its magnetic field bends the electron beam through a first angle The pole pieces of the first magnet may be specially shaped so that energy dispersion takes place along the beam path, resulting in an energy separation spectrum at the output of the first magnet. The field strength of this magnet is adjusted so that electrons of the required mean energy are transmitted.

The second magnet (M2), which may be described as a bending fine magnet, reverses the angle of bending action, that is to say it bends the electron beam through an equal and opposite (first) angle, from the first magnet and the field from its specially shaped pole pieces produces a focusing action in two orthogonal directions. In this region of the beam path, the widest energy separation occurs before beam convergence commences.

The converging beam from the second magnet is directed into the third magnet (M3), which also has specially shaped pole pieces. This magnet bends the beam through a second angle of over 90 degrees and completes the two-dimensional focusing action. The beam may be focused finally to an area of approximately 2 mm in diameter at the target. Optionally, the first angle is 44 degrees, and the second angle is 112 degrees, although it will be appreciated that other angles may be used.

The coils of all three magnets are water cooled. There are 2 coils per magnet. There is a thermal switch attached to each of the six coils and all six switches are wired in series to provide the bending magnet over-temperature circuit.

The bending magnet power supply units, PSUs, comprise two thyristor-controlled current-stabilized power supplies located in low voltage PSU area 13. The main supply (PSU1) contains a three-phase, full-wave, thyristor controlled bridge-rectifier and three LV PSU control PCBs. The secondary supply (PSU2) contains a single phase, full wave thyristor controlled bridge rectifier with a single control PCB.

The linac also comprises a voltage sensor 230, e.g. a voltmeter, at the bending magnet apparatus. The voltage sensor 230 may be comprised within the bending magnet power supply. The voltage sensor 230 is suitable for detecting, and is configured to detect, the actual voltage across the bending magnet assembly. The voltage sensor 230 is communicatively coupled with a device controller 240, and data relating to the bending magnet voltage is communicated to the device controller. The bending magnet voltage is measured by the voltage sensor and communicated to the device controller 240 with a particular frequency, for example the voltage sensor 230 may provide a voltage measurement to the device controller 240 every second.

The voltage sensor comprises means with which to communicate with the device controller 240. For example, the voltage sensor may comprise suitable processing circuitry and transmitting antennas. The voltage sensor is electronically and/or communicatively coupled to the device controller 240. The device controller 240 receives signals from the voltage sensor as they are generated, or produced, by the voltage sensor. The device controller 240 is electronically and/or communicatively coupled to a device controller memory 245. The device controller 240 and device controller memory 245 may be configured to store signals generated by the voltage sensor. The generated signals from the voltage sensor comprise sensor data.

The device controller 240 is communicatively coupled to a central controller 270, for example via a network 250. The device controller 240 is configured to transmit, i.e. send, the sensor data to the central controller 270 to be stored on the central controller memory 275. The central memory 275 may comprise a number of different servers as part of a cloud storage solution. The central controller may be communicatively coupled to a plurality of radiotherapy devices via network 250, each of which are configured to transmit signals to the central controller 270 to be stored on central memory 275. Central controller 270 is adapted and configured to process received signals and store them in a database. Processing the signals may comprise, for example, calculating and storing daily averages of particular sensor data.

The radiotherapy device has a variety of other sensors (not shown), the signals and readings from which are communicated to the device controller 240. The signals may be stored in the device controller memory 245 and/or may be communicated via the network to the central controller 270. The data may be uploaded to the central controller 270 as it is generated, or may be stored on the device controller memory 245 in order to be uploaded as a batch upload, for example at regular time intervals. Alternatively, the data may be continuously gathered by the device controller 240, for example the sensor signals may be sampled every 4 seconds while the device is not delivering radiation, and data is uploaded if the data shows a particular variance from the previously uploaded data point. In a specific implementation, the data points are uploaded when there is a change of +/-0.04, and the device controller looks for a new data item once every 4 seconds while the device is not delivering radiation, and once every second while the linac is delivering radiation.

The data is stored in a database on central memory 275, which may comprise data from sensors, for example the data includes the voltage values as denoted by signals from the voltage sensor, the degree of rotation of the gantry, whether or not radiation is being delivered at a particular time and the dose rate and machine output as indicated by a dosimeter or monitor chamber, as well as the water temperature at various points around the water cooling system. These types of data are given to provide examples, and the skilled person will appreciate that a modem linac device may be configured to generate a wealth of data from a large variety of sensors.

The device controller 240 and central controller 270 are both also communicatively coupled to a remote controller 260. The remote controller 260 may access the central database, which stores information and data regarding a plurality of radiotherapy devices, through the database 250 and by using a suitable software platform. The remote controller 260 may also access the device controller 240 to obtain real-time information regarding a particular radiotherapy machine.

The device, central and remote controllers may each be described as a processor, computer, or computing device. The controllers may be connected (e.g., networked) to each other and/or to other machines in a Local Area Network (LAN), an intranet, an extranet, or the Internet. The controllers may each operate in the capacity of a server or a client machine in a client-server network environment, or as a peer machine in a peer-to-peer (or distributed) network environment. The controllers may each be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, respective controllers are illustrated, the term "controller" shall also be taken to include any collection of machines (e.g., computers) that individually or jointly execute a set (or multiple sets) of instructions to perform any one or more of the methodologies discussed herein.

The approaches of the present disclosure may be embodied on one or more of the device controller memory and the remote controller memory, or any other computer-readable medium. The medium may be a non-transitory computer-readable medium. The computer-readable medium carries computer-readable instructions arranged for execution upon a processor so as to make the controller / processor carry out any or all of the methods described herein. The term "computer-readable medium" as used herein refers to any medium that stores data and/or instructions for causing a processor to operate in a specific manner. Such storage medium may comprise non-volatile media and/or volatile media. Non-volatile media may include, for example, optical or magnetic disks. Volatile media may include dynamic memory. Exemplary forms of storage medium include, a floppy disk, a flexible disk, a hard disk, a solid state drive, a magnetic tape, or any other magnetic data storage medium, a CD-ROM, any other optical data storage medium, any physical medium with one or more patterns of holes, a RAM, a PROM, an EPROM, a FLASH-EPROM, NVRAM, and any other memory chip or cartridge.

In a linac device, it is important that the bending magnet function stably so that the beam is focussed correctly on the target, and so that the x-rays be emitted correctly from the target. The operating temperature of the bending magnet coils may rise with time through use, and this can be indirectly measured through measuring the bending magnet voltage. The temperature should be kept constant by virtue of the operation of the cooling assembly. The measured bending magnet voltage provides an indirect indication of the operating temperature of the bending magnet assembly, assuming the bending magnet assembly is receiving a stable input current, and the cooling assembly is functioning correctly.

A representation of a bending magnet is shown in Fig. 3. A bending magnet apparatus is shown and comprises bending coarse stage and a bending fine stage 3 pairs of coils including coils which make up both a bending coarse stage and a bending fine stage. The bending magnet apparatus comprises a bending fine flying lead 301, a magnet plate 302, on which a bracket 303 is mounted. Bending magnet coils 304 are mounted in the bending magnet assembly. The bending magnet assembly also comprises water manifolds 305, 307, from which extend cooling pipes. The cooling fluid flows around the bending magnet coils 304 in order to regulate the temperature of the bending magnet assembly during operation of the linac. The bending machine apparatus comprises coil retaining brackets 306 which hold the coils in place. The bending coarse stage comprises positive and negative power supply connections 308. Water is used as a cooling fluid, though other cooling fluids may be used.

The bending magnet assembly comprises a plurality of thermal cut-outs located at the bending coils. The thermal cut-outs operate to suspend the accelerator when an operating temperature cut-off threshold is reached.

The bending magnet assembly is powered by a pair of low voltage power supply units (LVPSU), and these comprise two thyristor-controlled current-stabilized power supplies, located in low voltage PSU. A PSU arrangement is shown in figure 4.

Figure 4 shows an arrangement for delivering power to the bending magnet assembly. All three magnets M1, M2 and M3are supplied by PSU 1, and PSU2 is a top-up supply for magnet 3 only. It is used to compensate for the fact that, unlike magnet M1 and magnet M2, the current in magnet M3 is not linearly related to its field strength. The bending coarse and bending fine set current values are applied to a control circuit board. The outputs from the board are control signals applied to the inputs of PSU 1 and PSU 2. The bending magnet coil currents may be monitored by respective PSU control PCBs, which detect the voltage across resistors (not shown) located in the current return path of each PSU. The signals are fed back to the central control system, and may be used to apply servo-control to the bending coarse and bending fine current values.

The bending magnet voltage is measured at the bending magnet assembly in order to assess the performance of the bending magnet assembly. The LV PSUs may comprise smoothing chokes (not shown), at which voltage testing can be conveniently carried out.

Accordingly, broadly speaking the first sensor or sensors may be configured to provide signals indicative of voltage at measurement locations in the bending magnet assembly. The sensors may comprise any number of possible sensors which are suitable to measure voltage, current or resistance.

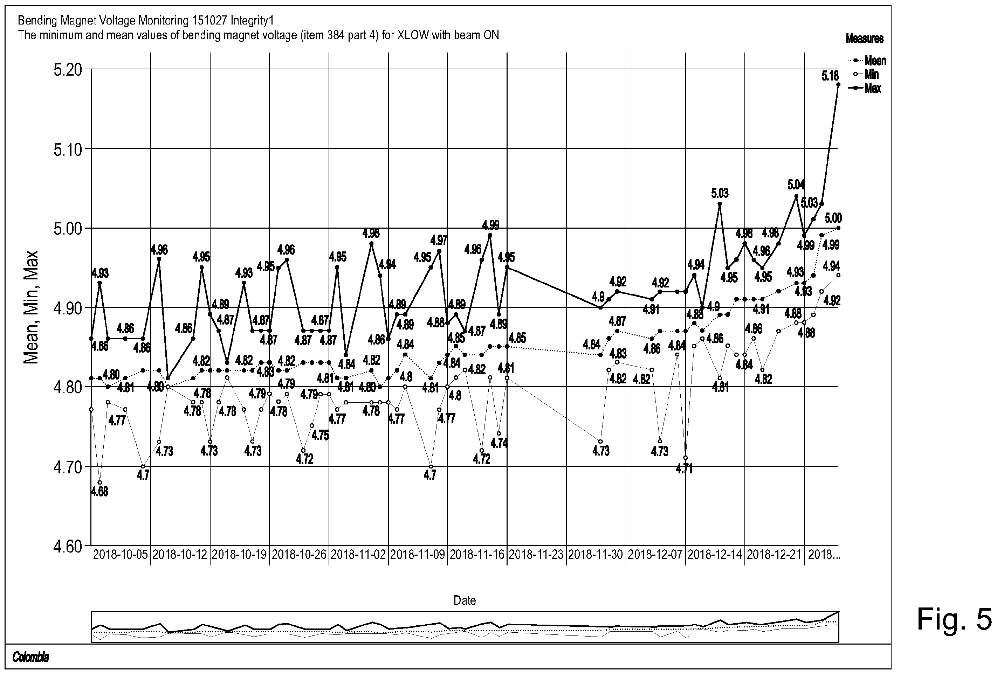

Figure 5 shows a graph generated using data from a database of sensor values as provided by sensors such as the sensors 230 depicted in figure 2. The graph shows time along the X axis and voltage along the Y axis. The unit of the y axis is a semi-arbitrary unit which is derived from the voltage values in the bending magnet assembly. It can be seen that the mean voltage recorded shows little variation prior to 2018-11-30, but then rises steadily until the most recently recorded data point.

The graph shows signals received from the bending magnet assembly as generated by the bending magnet voltage sensor. The graph demonstrates the type of signals which may be received from the device controller and which may be accessible by a remote controller. The line which is generally uppermost on the graph is the bending magnet voltage daily maximum reading. Each data point is the maximum value for a particular day, i.e. the maximum value for a particular date between a period from 00:00 to 23:59. The line which is generally lowermost on the graph is the bending magnet voltage daily minimum reading, with each data point being the minimum value that day. Finally, the line which is generally between the maximum and minimum lines is the daily average line. Each data point on this line is the average bending magnet voltage value for a particular day. In an implementation, it is this daily average value for the bending magnet voltage which is compared to a first threshold in order to determine whether a fault with the bending magnet assembly exists.

### Specific method

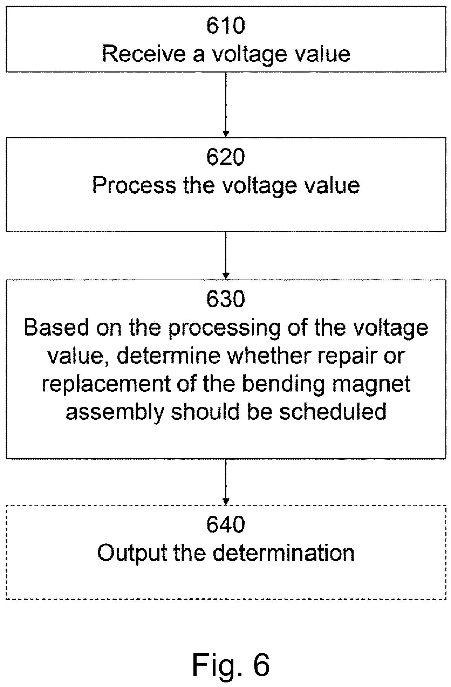

Reference is now made to the method depicted in the flowchart of figure 6. At step 610, a voltage value is received. The voltage value is derived from signals from the voltage sensor. As set out above, there is an optimal voltage value for a particular operation so as to achieve the correct beam bending, however the actual voltage supplied to the bending magnet assembly may vary slightly in the vicinity of this optimal value as the radiation beam is applied and because the current and/or the voltage supplied to the bending magnet assembly is controlled by a servomechanism. If the current is adjusted by the servomechanism, the voltage at the bending magnet assembly changes as the current is adjusted. The major variation in voltage from an optimal or mean value during normal operation is due to changes in coil temperature of the bending magnets. Therefore, the voltage measured by the voltage sensor 230 and received by the device controller 240 varies as the linac operates. The voltage value received at step 610 may be an average of signals or values received from the voltage sensor over a time period. In other words, the voltage value may be measurement of an average voltage received from the bending magnet assembly over the time period. Preferably, the time period is a day, such that the voltage value is a daily average of the voltage supplied to the bending magnet assembly. The method may further comprise deriving the voltage value by calculating or otherwise determining the average of the received signals from the voltage sensor 230 over the time period.

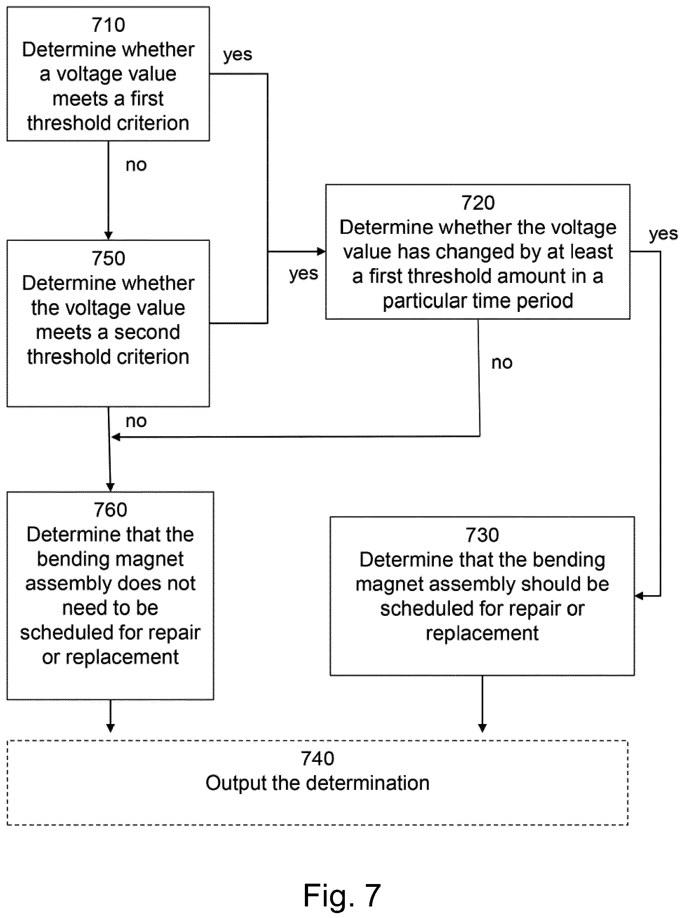

At step 620, the voltage value is processed. The processing of the voltage value is described in more detail in relation to figure 7, though in a preferred embodiment the processing of the voltage value comprises determining whether the voltage value meets at least one threshold criterion and determining whether the voltage value has changed by a threshold amount or more in a particular time period.

At step 630, based on the processing of the voltage value, it is determined whether repair or replacement of the bending magnet assembly should be scheduled. In an example, it is determined that repair or replacement should be scheduled if the determinations at step 620 described above are positive, i.e. if the voltage value meets at least one threshold criterion and the voltage value has changed by at least the threshold amount in a particular time period.

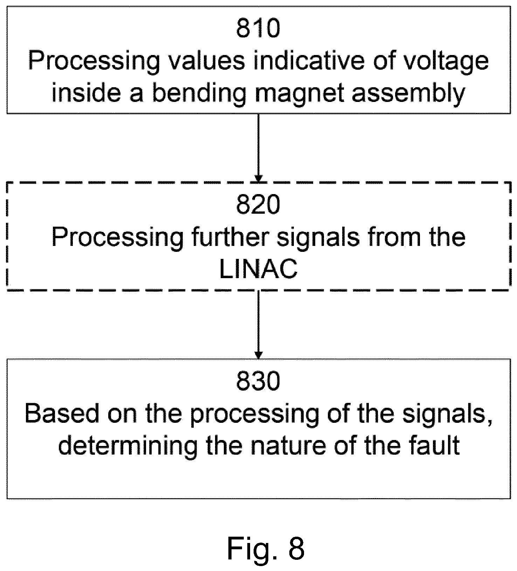

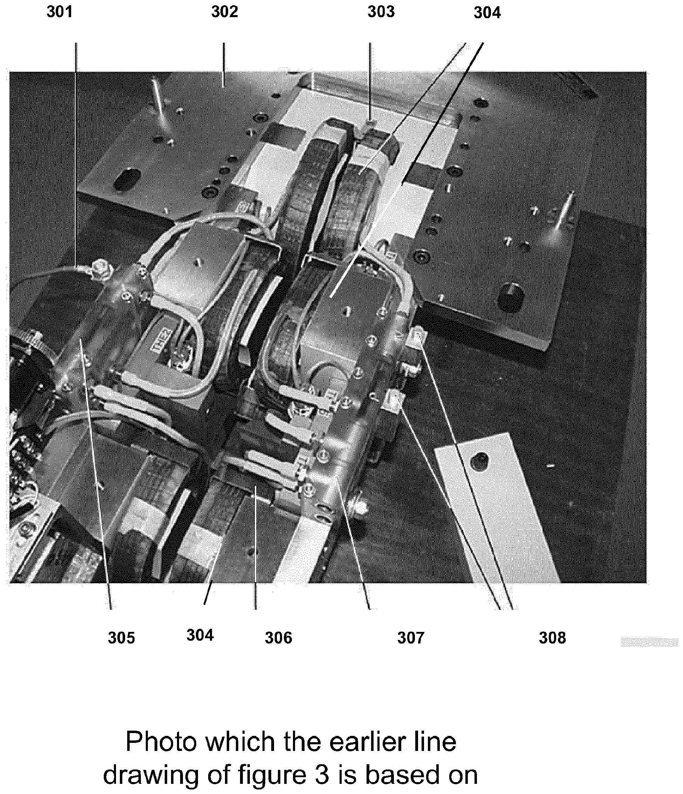

By way of a specific example of steps 610, 620, and 630, signals relating to the voltage supplied to the bending magnets are received at the device controller 240 from the voltage sensor 230 and are communicated to the central controller 270 to be stored in the central memory 275. The central controller 270 receives a daily average of the voltage by calculating, or determining, the daily average based on the received voltage values. The central controller also receives a notification of the set bending magnet voltage. It is determined whether the difference between the set bending magnet voltage and the actual bending magnet voltage measured by the sensor exceeds a first threshold. For example, if a difference between the set value and the actual value is more than 0.2V, then it may be determined that repair or replacement of the bending magnet assembly is required, or that further analysis might be triggered. The daily average of the voltage may also be processed; in particular it is determined whether the average voltage for a particular radiation energy (for example the XLOW) has increased by more than 0.1V or more in the last 30 days. The daily average of the voltage may also be compared to the daily minimum voltage, and where a difference between the two values exceeds a threshold for a predetermined time period, it may be determined that the bending magnet should be repaired or replaced. For example, where a difference of more than 0.15V occurs 4 times (i.e. on four days) or more in a period of four weeks, a repair determination may be triggered. Based on the processing of the average voltage values, it is determined whether repair or replacement of the bending magnet assembly should be scheduled. For example, if the above-described determinations are positive, it is determined that the bending magnet assembly should be repaired or replaced. Alternatively, it may be determined that a secondary analysis step is required, described further below in relation to figure 8.

At step 640, the determination at step 630 of whether the repair or replacement should be scheduled is outputted. This step is optional. Outputting the determination may comprise displaying an indication of the determination on a display screen. In response to this indication, the repair or replacement of the bending magnet assembly can be scheduled at the next available service point, or at a convenient time for the machine owner, for example during planned machine down time. Outputting the determination may also comprise issuing an alert or notification detailing the determination to a field service engineer. The alert may comprise information regarding how to test the bending magnet assembly for faults and may also comprise information detailing how to replace the bending magnet assembly, or how to repair the bending magnet assembly for example by chemical flushing. Outputting the determination may also comprise issuing a communication, alert, or otherwise contacting the owner of the radiotherapy device in order to inform them of the issue and to schedule the repair or replacement.

In an example, once the prediction criteria described herein and set out in the flowchart of figure 7 have been met, a report is generated and an alert is raised. This alert is then sent to the relevant service team and they will plan a repair or replacement, typically within the next 3-6 weeks or to align with a planned maintenance activity.

In a preferred embodiment, the device controller 240 receives signals from the voltage sensor and transmits them to the remote controller 260. The central controller 270 receives the signals and then derives the voltage value, for example by calculating the average voltage value from the signals over a time period. The average voltage value is stored on the central controller memory 270. Processing of the voltage value then takes place when the remote controller 260 accesses the central memory 275 via the network 250. However, at least some of the steps, and in some examples all of the steps, displayed in figure 6 may be performed on the device controller 240. It will be appreciated that any combination of the device controller 260 central controller 270 and remote controller 260 may be used to perform the disclosed methods.

Reference is now made to the flowchart of figure 7. The flowchart shows steps 620, 630 and the optional step of 640 in greater detail, and in particular shows the processing of the voltage value in greater detail. At step 710, it is determined whether the received or derived voltage value meets a first threshold criterion. In a particular example, checking whether the first criterion is met comprises determining that the voltage value, e.g. the mean bending magnet voltage value for a particular day, exceeds a first threshold value, for example a first threshold value determined by a nominal operating voltage value, or "set" bending magnet voltage. If so, the criterion is met. In a particular example, it is determined whether the mean bending magnet voltage is different from the set value by more than 0.2V.

If the voltage value meets the first threshold criterion, then the process proceeds to step 720. At step 720, it is determined whether the voltage value has changed by at least a first threshold amount in a particular time period. The first threshold amount can be described as a first particular amount. In a particular example, the first threshold amount is 0.1V and the particular time period is 30 days, such that it is determined whether the mean bending magnet voltage has increased by 0.1V or more in the last 30 days. If so the criterion is met.

If the voltage value has changed by the threshold amount in the time period, then it is determined, at step 730, that the bending magnet assembly should be scheduled for repair or replacement. This determination may be outputted at step 740 in a manner similar to the outputting at step 640.

If at step 720 it is determined that the voltage value has not changed by the first or second threshold amount, as appropriate, in the particular time period, then the replacement/ repair criteria are not fulfilled and it is determined at step 760 that the bending magnet assembly should not be repaired or replaced.

If at step 710 it is determined that the voltage value does not meet a first threshold criterion, e.g. the difference between the actual and the set bending magnet voltage is not more than 0.2V, then at step 750 it is determined whether the voltage value meets a second threshold criterion. In an example, checking or determining whether the second threshold criterion is met may comprise determining whether the voltage value is above a second threshold value, for example a second average voltage value. In a particular example, checking the second threshold criterion is met comprises determining that the voltage value, e.g. the mean bending magnet voltage value for a particular day, is above a second threshold value.

Step 750 may alternatively or additionally comprise determining that a difference between the average voltage value for a given day and the lowest measured value for a day is greater than a threshold value, for example 0.15V, and the determination may comprise that this has occurred consistently over a number of days in a recorded period. For example, it may be determined at step 750 that the difference between the lowest and mean recordings has exceeded 0.15V for five days of the previous seven, or ten days of the previous twenty, and this may be deemed as meeting the second threshold criterion.

If the voltage value meets the second threshold criterion or criteria, then the process proceeds to step 720.

If the voltage value has changed by the threshold amount in the time period, then it is determined, at step 730, that the bending magnet assembly should be scheduled for repair or replacement. This determination may be outputted at step 740 in a manner similar to the outputting at step 640.

In an alternative to the arrangement shown in Fig. 7, all three of steps 710, 720, and 750 may require a "yes" determination in order to make a determination that the bending magnet assembly comprises a fault and should be scheduled for repair. Equally, step 730 may include the further step of performing a secondary analysis of the voltage values received, as will be explained below.

It has been noted by the inventors that, as the bending magnet assembly in a linac reaches the end of its useful life, the rate of change of the optimal voltage increases. In other words, the voltage which must be supplied to the bending magnet assembly for a particular linac energy in order to attempt to obtain optimal beam bending increases more rapidly as the bending magnet assembly reaches the end of its life. Using thresholds relating to whether the voltage value has changed by a particular amount in a time period, i.e. which relate to the rate of change of the voltage value, allows calibration errors to be accounted for, and ensures that only devices which are accelerating toward failure are flagged for repair or replacement. The use of thresholds relating to the rate of change of the bending magnet voltage help to ensure that unnecessary service visits and/or repairs and replacements are not occasioned.

In a specific example, determining whether the first criterion is met comprises checking that the difference between the actual bending magnet assembly voltage and the set voltage is more than 0.2V, and determining whether the second criterion is met comprises checking that the difference between the lowest bending magnet voltage and the mean or average bending magnet voltage is greater than 0.15V, consistently, or regularly over the predetermined time period, which may be a week or 30 days or similar. With reference to the graph of figure 5, it can be seen that the mean bending magnet voltage has risen steadily, and that on a number of occasions the difference between the minimum value and the mean value has exceeded 0.15V, and that the bending magnet voltage is above the set bending magnet voltage.

Once the three criteria are met, further analysis can be triggered as part of a fault notification.

The methods of the present disclosure may be performed on a regular basis, for example daily, in order to continuously monitor whether the bending magnet assembly of a radiotherapy device should be repaired or replaced, or at least scheduled for repair or replacement.

It will be appreciated that while a specific order of steps is set out in figure 7, these steps may be performed in a different order.

It will be appreciated that while reference is made to the XLOW voltage, i.e. the voltage supplied to the bending magnet assembly for the lowest configured energy of the linac, the voltage while the linac is operating at any particular energy may be used. For machines manufactured by the applicant, XLOW is typically 6MV, and the set bending magnet voltage is approximately 4.00V, and 6MV is used more than any other energy. In fact, around 85% of all clinical use is with 6MV. The advantage of using the voltage at the XLOW energy is therefore that this is the energy most used by the linac machine and hence monitoring the voltage at this energy will produce the most data items for analysis. The set bending magnet voltage can be used as a reference value in determining whether the measured bending magnet voltage differs from it by more than a reference difference value.

Methods of the present disclosure may be described as computer-implemented methods which allow a determination that a component of a radiotherapy device, namely a bending magnet assembly in a clinical linac device, should be scheduled for repair or replacement. Repair of the bending magnet assembly may comprise flushing the cooling tubes to remove a blockage. The present techniques therefore describe a cost-effective, efficient and predictive method of preventing a fault. It has to date proved impossible to determine the condition of components of an in-service bending magnet assembly in order to predict when maintenance should be performed. The present approach provides cost and time savings over the prior methods because servicing and maintenance in the form of repair and replacement of the bending magnet assembly is only performed when needed, and downtime of the device is reduced due to reducing or eliminating entirely the number of safety interrupts usually associated with a degrading cooling system, and also by eliminating time spent diagnosing the problem before repair is effected.

Reference is now made to the flowchart of figure 8. The method corresponds with the methods depicted in figures 6 and 7, above, however the flowchart of figure 8 details the tasks which are performed by the remote controller 245, otherwise deemed the remote access controller. The steps of the flowchart in Figure 8 may be carried out where a fault condition is identified as described above in relation to figures 6 and 7.

At step 810, the voltage values are processed. As described above, the values are derived from signals received from the first sensor and may be, for example, respective averages of signals received from the first sensor. The values may be determined by any of the device controller, the central controller, or the remote controller. Processing the values further comprises reviewing voltage values at a higher resolution than carried out in the methods described above, which may use the daily average voltage values.

In an implementation, to determine a blockage in the cooling system, a review of voltage values for a single day of operation may be processed. It can be determined that the bending magnet voltage increases throughout the day's operation, and therefore that the cooling system is not functioning correctly. A fault can be determined as part of the cooling system. In this way, the processing of the values indicative of the voltage in the bending magnet assembly can comprise a review at a first data resolution, using for example the daily average values, and where the threshold criteria are met, further processing of the values at a second data resolution, for example looking at the development of bending magnet voltages across a single day.

In an optional step 820, bending magnet voltage data is reviewed in correspondence with further signals obtained from the LINAC to determine a nature of the fault. In an implementation, an additional step of checking whether the signals from either the first and/or the second sensor, or values derived from these signals, vary with the gantry rotation angle. In other words, an additional step of checking whether there appears to be any correlation between bending magnet voltage, and the angle to which the gantry has been rotated. For example, if there is a correlation, e.g. if the voltage values do vary with gantry angle by more than a threshold variance, then it is determined that the issue is not associated with the bending magnet assembly cooling. This is because it has been determined that bending magnet assembly cooling issues are not dependent on the angle to which the gantry has been rotated. In general, the processing of further signals from the linac can take place at the second resolution, for finer analysis. Any available bending magnet voltage data, including any and all data types discussed herein, may be further investigated with respect to the gantry rotation value. In a simple example, gantry rotation angles can be split into 'bins', e.g. four bins between 0° and 90°, between 90° and 180°, between 180° and 270°, and between 270° and 360°. Voltage signals received while the gantry was at these angles are processed. For example, an average bending magnet voltage value can be determined for each bin. If any of the average values vary from each other by more than a threshold variance, it suggests that the voltage level is dependent on the angle to which the gantry has been rotated. With a bending magnet assembly cooling issue, it is not expected that gantry rotation will influence the voltage. This is in contrast with other issues which may present themselves, for example movement of components causing a partial short, which do show fluctuations in voltage values which are angle independent.

The instantiation of secondary or further processing of the voltage values or further signals from the linac at the second data resolution, once threshold criteria have been met at a lower resolution means that the wealth of signal data being collected from the sensors at the linac only need be analysed when necessary.

These steps may be performed at any of the controllers, but in a preferred embodiment is performed at the remote controller.

At step 830, based on the processing of the signals or further signals, it is determined whether or not the nature of the fault is associated with, e.g. caused by, the cooling system of the bending magnet assembly. As with the method of figure 7, there may be an additional step of outputting the nature of the fault in the radiotherapy device. Outputting the nature of the fault may comprise displaying an indication of the nature of the fault on a display screen. For example, the display may indicate that the fault either is, or isn't, associated with the bending magnet assembly. Alternatively or additionally, the method may comprise automatically issuing an alert identifying the nature of the fault to a field service engineer. The alert may comprise information identifying the machine at fault as well as the nature of the fault, along with information on recommended approaches to addressing and fixing the fault.

Methods of the present disclosure allow a determination that the nature of a fault in a radiotherapy device, for example a clinical LINAC device, is associated with the bending magnet assembly. Prior techniques can identify that a LINAC machine has a fault, but cannot provide any further information which may assist a field service engineer. In contrast, the present methods allow both the identification of a fault, and a determination that the nature of the fault is associated with the bending magnet assembly, for example the bending magnet assembly cooling. The present techniques therefore describe a cost-effective and efficient method of determining a fault.

The approaches in the prior art to date to address a fault has been to send field service engineers to carry out diagnostic tests on the machine. As detailed herein, this prior technique is time consuming, inefficient and expensive. It has until now proved difficult to further specify the nature of a fault with the bending magnet assembly for a number of reasons. The bending magnet voltage can be affected by a large number of factors, for example due to temperature increases caused by an inefficient or malfunctioning cooling system, and electrical connection faults. Further, several different possible faults may appear to manifest themselves in similar or identical ways, for example by raising the bending magnet voltage. Possible components which could be degrading, or operating at non-optimal performance, and which may cause faults that are broadly associated with the bending magnet assembly include one or more of the following components: the low voltage power supply units, the cooling system, and loose/flexing connections between the components which form the bending magnet assembly. The issue is not that these components may degrade to a level which may compromise patient safety, as the bending magnet assembly is monitored to ensure patient safety at all times. However, once it has been determined that a fault exists which is broadly associated with the bending magnet assembly it has to date proved difficult to identify which component is responsible.

By indicating to the field service engineer before he or she attends the machine that the bending magnet assembly is likely to be at fault, fewer ineffective solutions are attempted and fewer maintenance visits are needed.

At least some of the present techniques generally involve processing signals from sensors which provide signals indicative of operating conditions at the bending magnet assembly and using the variation in signals to determine that an issue is likely associated with the bending magnet assembly of the LINAC device.

The above implementations have been described by way of example only, and the described implementations and arrangements are to be considered in all respects only as illustrative and not restrictive. It will be appreciated that variations of the described implementations and arrangements may be made without departing from the scope of the invention as defined in the appended claims.

## Claims

1. A method of determining the nature of a fault in a radiotherapy device comprising a linear accelerator, the radiotherapy device being configured to provide therapeutic radiation to a patient, the radiotherapy device comprising:
a bending magnet assembly (208) arranged to direct a beam of electrons toward a target to produce said radiation; and
a first sensor (230), configured to provide signals indicative of voltage at a first region of the bending magnet assembly; the method comprising:
receiving (610) voltage values derived from the signals from the first sensor;
processing (620) the voltage values; wherein processing the voltage values comprises
determining whether the voltage values meet at least one threshold criterion; and
based on the processing of the signals, determining (630) whether the nature of the fault is associated with the bending magnet,
wherein determining whether the voltage values meet the at least one threshold criterion further comprises: determining whether the voltage values have changed by at least a threshold amount in a particular time period.

2. The method of claim 1, wherein determining whether the voltage values meet the at least one threshold criterion comprises
determining that a difference between a particular voltage value and a reference value has exceeded a reference difference value.

3. The method of any preceding claim, wherein determining whether the voltage values meet the at least one threshold criterion further comprises:
determining that the particular voltage value has fallen below a threshold voltage value.

4. The method of any preceding claim, wherein the processing the voltage values comprises:
determining whether the voltage values meet the at least one threshold criterion at a first data resolution, and
in response to the at least one threshold criterion being met,
processing the voltage values at a second data resolution.

5. The method of claim 4, wherein the second data resolution is higher than the first data resolution.

6. The method of any preceding claim, the radiotherapy device further comprising:
a second sensor configured to provide operating signals indicative of an operating parameter of the radiotherapy device, the method further comprising:
processing values derived from the operating signals in response to the determination that the voltage value meets the threshold criterion,
wherein determining the nature of the fault comprises determining a correlation between the voltage values and the operating signals.

7. The method of claim 6 wherein the operating parameter is one of:
a movement of a gantry of the radiotherapy device
a rotation angle of the gantry of the radiotherapy device;
a temperature at a wiring location of the radiotherapy device;
an operational state of the radiotherapy device; and
a beam energy of the beam of electrons.

8. The method of any preceding claim, further comprising outputting the nature of the fault in the radiotherapy device.

9. The method of claim 7, wherein outputting the nature of the fault comprises at least one of displaying an indication of the nature of the fault on a display screen and/or issuing an alert identifying the nature of the fault to a field service engineer.

10. The method of any preceding claim, wherein the radiotherapy device further comprises a rotatable gantry to which a radiation source is attached, the radiation source being configured to provide the therapeutic radiation to a patient, wherein processing the first value and the operating parameter further comprises determining that the first signal and or the operating parameter are not dependent on a rotation angle of the gantry.

11. A computer-readable medium comprising computer-executable instructions which, when executed by a processor, cause the processor to perform the method of any preceding claim.

12. A system comprising:
a remote controller (260);
a central controller (270); and
a radiotherapy device;
wherein the remote controller is communicatively coupled to the central controller via a network, the central controller configured to receive data from the radiotherapy device, the radiotherapy device comprising a linear accelerator, the radiotherapy device being configured to provide therapeutic radiation to a patient, and the radiotherapy device comprising:
a bending magnet assembly (208) arranged to direct a beam of electrons toward a target to produce said radiation; and
a first sensor (230), the first sensor being configured to provide signals indicative of voltage at a first region of the bending magnet assembly;
the remote controller further being coupled to a computer-readable medium comprising computer-executable instructions which, when executed by the remote controller, causes the remote controller to:
request and receive, from the central controller, a first value derived from signals from the first sensor, and
perform the method of any of claims 1 to 10.

## Patentansprüche

1. Verfahren zum Bestimmen der Art eines Fehlers in einer Strahlentherapievorrichtung, die einen Linearbeschleuniger umfasst, wobei die Strahlentherapievorrichtung ausgestaltet ist, um einem Patienten therapeutische Strahlung bereitzustellen, wobei die Strahlentherapievorrichtung Folgendes umfasst:
eine Ablenkmagnetbaugruppe (208), die dazu angeordnet ist, einen Elektronenstrahl auf ein Ziel zu richten, um die Strahlung zu produzieren; und
einen ersten Sensor (230), der ausgestaltet ist, um Signale bereitzustellen, die eine Spannung in einer ersten Region der Ablenkmagnetbaugruppe angeben; wobei das Verfahren Folgendes umfasst:
Empfangen (610) von Spannungswerten, die von den Signalen von dem ersten Sensor abgeleitet sind;
Verarbeiten (620) der Spannungswerte; wobei das Verarbeiten der Spannungswerte Bestimmen umfasst, ob die Spannungswerte mindestens ein Schwellenwertkriterium erfüllen; und
basierend auf dem Verarbeiten der Signale Bestimmen (630), ob die Art des Fehlers mit dem Ablenkmagneten in Zusammenhang steht,
wobei das Bestimmen, ob die Spannungswerte das mindestens eine Schwellenwertkriterium erfüllen, ferner Folgendes umfasst: Bestimmen, ob sich die Spannungswerte in einem bestimmten Zeitraum um mindestens einen Schwellenbetrag geändert haben.

2. Verfahren nach Anspruch 1, wobei das Bestimmen, ob die Spannungswerte das mindestens eine Schwellenwertkriterium erfüllen, Folgendes umfasst: Bestimmen, dass eine Differenz zwischen einem bestimmten Spannungswert und einem Referenzwert einen Referenzdifferenzwert überschritten hat.

3. Verfahren nach einem der vorhergehenden Ansprüche, wobei das Bestimmen, ob die Spannungswerte das mindestens eine Schwellenwertkriterium erfüllen, ferner Folgendes umfasst:
Bestimmen, dass der bestimmte Spannungswert unter einen Schwellenspannungswert gefallen ist.

4. Verfahren nach einem der vorhergehenden Ansprüche, wobei das Verarbeiten der Spannungswerte Folgendes umfasst:
Bestimmen, ob die Spannungswerte das mindestens eine Schwellenwertkriterium bei einer ersten Datenauflösung erfüllen, und
in Reaktion darauf, dass das mindestens eine Schwellenwertkriterium erfüllt wird,
Verarbeiten der Spannungswerte bei einer zweiten Datenauflösung.

5. Verfahren nach Anspruch 4, wobei die zweite Datenauflösung höher als die erste Datenauflösung ist.

6. Verfahren nach einem der vorhergehenden Ansprüche, wobei die Strahlentherapievorrichtung ferner Folgendes umfasst:
einen zweiten Sensor, der ausgestaltet ist, um Betriebssignale bereitzustellen, die einen Betriebsparameter der Strahlentherapievorrichtung angeben, wobei das Verfahren ferner Folgendes umfasst:
Verarbeiten von Werten, die von den Betriebssignalen abgeleitet sind, in Reaktion auf die Bestimmung, dass der Spannungswert das Schwellenwertkriterium erfüllt,
wobei das Bestimmen der Art des Fehlers Bestimmen einer Korrelation zwischen den Spannungswerten und den Betriebssignalen umfasst.

7. Verfahren nach Anspruch 6, wobei der Betriebsparameter eines von Folgendem ist:
eine Bewegung einer Gantry der Strahlentherapievorrichtung;
ein Rotationswinkel der Gantry der Strahlentherapievorrichtung;
eine Temperatur an einer Verdrahtungsstelle der Strahlentherapievorrichtung;
ein Betriebszustand der Strahlentherapievorrichtung und eine Strahlenergie des Elektronenstrahls.

8. Verfahren nach einem der vorhergehenden Ansprüche, ferner umfassend Ausgeben der Art des Fehlers in der Strahlentherapievorrichtung.

9. Verfahren nach Anspruch 7, wobei das Ausgeben der Art des Fehlers mindestens eines von Anzeigen einer Angabe der Art des Fehlers auf einem Anzeigebildschirm und/oder Ausgeben eines Alarms, der die Art des Fehlers identifiziert, an einen Außendiensttechniker umfasst.

10. Verfahren nach einem der vorhergehenden Ansprüche, wobei die Strahlentherapievorrichtung ferner eine drehbare Gantry umfasst, an der eine Strahlungsquelle befestigt ist, wobei die Strahlungsquelle ausgestaltet ist, um einem Patienten die therapeutische Strahlung bereitzustellen, wobei das Verarbeiten des ersten Wertes und des Betriebsparameters ferner Bestimmen umfasst, dass das erste Signal und/oder der Betriebsparameter nicht von einem Rotationswinkel der Gantry abhängig sind.

11. Computerlesbares Medium, umfassend computerausführbare Anweisungen, die bei Ausführung durch einen Prozessor bewirken, dass der Prozessor das Verfahren nach einem der vorhergehenden Ansprüche durchführt.

12. System, umfassend:
eine Fernbedienung (260);
eine Zentralsteuerung (270); und
eine Strahlentherapievorrichtung;
wobei die Fernbedienung kommunikativ über ein Netzwerk an die Zentralsteuerung gekoppelt ist, wobei die Zentralsteuerung ausgestaltet ist, um Daten von der Strahlentherapievorrichtung zu empfangen, wobei die Strahlentherapievorrichtung einen Linearbeschleuniger umfasst, die Strahlentherapievorrichtung ausgestaltet ist, um einem Patienten therapeutische Strahlung bereitzustellen, und wobei die Strahlentherapievorrichtung Folgendes umfasst:
eine Ablenkmagnetbaugruppe (208), die dazu angeordnet ist, einen Elektronenstrahl auf ein Ziel zu richten, um die Strahlung zu produzieren; und
einen ersten Sensor (230), wobei der erste Sensor ausgestaltet ist, um Signale bereitzustellen, die eine Spannung in einer ersten Region der Ablenkmagnetbaugruppe angeben;
wobei die Fernbedienung ferner an ein computerlesbares Medium gekoppelt ist, das computerausführbare Anweisungen umfasst, die, wenn sie durch die Fernbedienung ausgeführt werden, die Fernbedienung zu Folgendem veranlassen:
Anfordern und Empfangen eines ersten Wertes, der von Signalen von dem ersten Sensor abgeleitet ist, von der Zentralsteuerung; und Durchführen des Verfahrens nach einem der Ansprüche 1 bis 10.

## Revendications

1. Procédé de détermination de la nature d'un défaut dans un dispositif de radiothérapie comprenant un accélérateur linéaire, le dispositif de radiothérapie étant conçu pour fournir un rayonnement thérapeutique à un patient, le dispositif de radiothérapie comprenant :
un ensemble aimant de courbure (208) agencé pour diriger un faisceau d'électrons vers une cible pour produire ledit rayonnement ; et
un premier capteur (230), conçu pour fournir des signaux indiquant une tension au niveau d'une première région de l'ensemble aimant de courbure ; le procédé comprenant :
la réception (610) de valeurs de tension dérivées des signaux provenant du premier capteur ;
le traitement (620) des valeurs de tension ; le traitement des valeurs de tension comprenant la détermination que les valeurs de tension respectent ou non au moins un critère seuil ; et
sur la base du traitement des signaux, la détermination (630) que la nature du défaut est associée ou non à l'aimant de courbure,
dans lequel la détermination que les valeurs de tension respectent ou non l'au moins un critère seuil comprend en outre : la détermination que les valeurs de tension ont varié ou non d'au moins une quantité seuil dans un laps de temps particulier.

2. Procédé selon la revendication 1, dans lequel la détermination que les valeurs de tension respectent ou non l'au moins un critère seuil comprend la détermination qu'une différence entre une valeur de tension particulière et une valeur de référence a dépassé ou non une valeur de différence de référence.

3. Procédé selon une quelconque revendication précédente, dans lequel la détermination que les valeurs de tension respectent ou non l'au moins un critère seuil comprend en outre :
la détermination que la valeur de tension particulière a chuté ou non en dessous d'une valeur de tension seuil.

4. Procédé selon une quelconque revendication précédente, dans lequel le traitement des valeurs de tension comprend :
la détermination que les valeurs de tension respectent ou non l'au moins un critère seuil à une première résolution de données, et
en réponse au fait que l'au moins un critère seuil est respecté,
le traitement des valeurs de tension à une deuxième résolution de données.

5. Procédé selon la revendication 4, dans lequel la deuxième résolution de données est supérieure à la première résolution de données.

6. Procédé selon une quelconque revendication précédente, le dispositif de radiothérapie comprenant en outre :
un deuxième capteur conçu pour fournir des signaux de fonctionnement indiquant un paramètre de fonctionnement du dispositif de radiothérapie, le procédé comprenant en outre :
le traitement de valeurs dérivées des signaux de fonctionnement en réponse à la détermination que la valeur de tension respecte le critère seuil,
dans lequel la détermination de la nature du défaut comprend la détermination d'une corrélation entre les valeurs de tension et les signaux de fonctionnement.

7. Procédé selon la revendication 6 dans lequel le paramètre de fonctionnement en est un parmi :
un mouvement d'un portique du dispositif de radiothérapie ;
un angle de rotation du portique du dispositif de radiothérapie ;
une température à l'emplacement d'un câblage du dispositif de radiothérapie ;
un état de fonctionnement du dispositif de radiothérapie ; et
une énergie de faisceau du faisceau d'électrons.

8. Procédé selon une quelconque revendication précédente, comprenant en outre la transmission de la nature du défaut dans le dispositif de radiothérapie.

9. Procédé selon la revendication 7, dans lequel la transmission de la nature du défaut comprend au moins une opération parmi l'affichage d'une indication de la nature du défaut sur un écran d'affichage et/ou la délivrance d'une alerte identifiant la nature du défaut à un ingénieur de maintenance.

10. Procédé selon une quelconque revendication précédente, dans lequel le dispositif de radiothérapie comprend en outre un portique rotatif auquel une source de rayonnement est fixée, la source de rayonnement étant conçue pour fournir le rayonnement thérapeutique à un patient, dans lequel le traitement de la première valeur et du paramètre de fonctionnement comprend en outre la détermination que le premier signal et/ou le paramètre de fonctionnement ne dépendent pas d'un angle de rotation du portique.

11. Support lisible par ordinateur comprenant des instructions exécutables par ordinateur qui, lorsqu'elles sont exécutées par un processeur, conduisent le processeur à effectuer le procédé d'une quelconque revendication précédente.

12. Système comprenant :
un dispositif de commande à distance (260) ;
un dispositif de commande central (270) ; et
un dispositif de radiothérapie ;
dans lequel le dispositif de commande à distance est couplé de façon communicante au dispositif de commande central par le biais d'un réseau, le dispositif de commande central étant conçu pour recevoir des données provenant du dispositif de radiothérapie, le dispositif de radiothérapie comprenant un accélérateur linéaire, le dispositif de radiothérapie étant conçu pour fournir un rayonnement thérapeutique à un patient, et le dispositif de radiothérapie comprenant :
un ensemble aimant de courbure (208) agencé pour diriger un faisceau d'électrons vers une cible pour produire ledit rayonnement ; et
un premier capteur (230), le premier capteur étant conçu pour fournir des signaux indiquant une tension au niveau d'une première région de l'ensemble aimant de courbure ;
le dispositif de commande à distance étant en outre couplé à un support lisible par ordinateur comprenant des instructions exécutables par ordinateur qui,
lorsqu'elles sont exécutées par le dispositif de commande à distance, conduisent le dispositif de commande à distance à :
demander et recevoir, depuis le dispositif de commande central, une première valeur dérivée de signaux provenant du premier capteur ; et effectuer le procédé de l'une quelconque des revendications 1 à 10.
